# EUROPEAN PATENT SPECIFICATION

(11) **EP 0 689 586 B1**
(45) Date of publication and mention of the grant of the patent: **02.05.2007**
(21) Application number: 94909839.6
(22) Date of filing: 22.02.1994
(51) Int. Cl.: C12N 5/00, C12N 5/10, C12N 15/49, A61K 38/00, A61K 39/21, A61K 48/00

(54) **VPR FUNCTION AND ACTIVITY**
FUNKTION UND AKTIVITÄT DES VIRALEN PROTEINS R (VPR)
ACTIVITE ET FONCTION DE LA PROTEINE VIRALE R

(30) Priority: 19.02.1993 US 19601; 15.12.1993 US 167608
(43) Date of publication of application: 03.01.1996
(73) Proprietor: Weiner, David B., Merion, PA 19066 (US); Levy, David Nathan, Boston, MA 02215 (US); Refaeli, Yosef, Boston, MA 02135 (US)
(72) Inventor: Weiner, David B., Merion, PA 19066 (US); Levy, David Nathan, Boston, MA 02215 (US); Refaeli, Yosef, Boston, MA 02135 (US)
(74) Representative: Hallybone, Huw George
(86) International application number: PCT/US1994/002191
(87) International publication number: WO 1994/019456

(56) References cited:
- WO-A-90/15875
- US-A- 5 001 230
- VACCINES (COLD SPRING HARBOR), 93. MODERN APPROACHES TO NEW VACCINES INCLUDING PREVENTION OF AIDS; TENTH ANNUAL MEETING, September 1992, COLD SPRING HARBOR,NEW YORK,USA, pages 243-249, XP000673175 LEVY ET AL: "HIV REGULATORY GENE FUNCTION ANALYSIS IN A RHABDOMYOSARCOMA CELL LINE"
- JOURNAL OF VIROLOGY, vol. 63, no. 9, September 1989, pages 4110-4114, XP002031071 OGAWA ET AL: "MUTATIONAL ANALYSIS OF THE HUMAN IMMUNODEFICIENCY VIRUS VPR OPEN READING FRAME"
- AIDS RESEARCH AND HUMAN RETROVIRUSES, vol. 7, no. 2, February 1991, page 177 XP002031072 HATTORI ET AL: "THE VPR GENE OF HIV-2 IS A TRANSACTIVATOR NECESSARY FOR VIRAL EXPRESSION IN PRIMARY MACROPHAGES"
- ABSTRACTS AND SELECTED PAPERS FROM THE 1993 ANNUAL MEETING OF THE LABORATORY OF TUMOR CELL BIOLOGY, 22 - 28 August 1993, BETHESDA, MARYLAND, page S92 XP002031073 LEVY ET AL: "INDUCTION OF CELL DIFFERENTIATION BY HIV-1 VPR" & AIDS RESEARCH AND HUMAN RETROVIRUSES, vol. 10, no. S1, August 1994,
- Journal of Cellular Biochemistry, Supplement 18B, issued 21 January - 13 February 1994, REFAELI et al., "Recombination HIV-1 Vpr Protein Induces Cellular Differentiation In Vitro", page 140, see abstract J 262.
- International Conference on AIDS (Canada), Volume 5, issued 4-9 June 1989, JANEL et al., "Localization of the VPR Gene Product in SIVmac Infected Cells", page 523, see Abstract T.C.O. 45.
- AIDS Research and Human Retroviruses, Volume 6, Number 11, issued 1990, YUAN et al., "Human Immunodeficiency Virus VPR Gene Encodes a Virion-Associated Protein", pages 1265-1271, see entire article.
- Journal of Virology, Volume 64, Number 11, issued November 1990, YU et al., "Open Reading Frame VPR of Simian Immunodeficiency Virus Encodes a Virion-Associated Protein", pages 5688-5693, see entire article.
- MUENCH M.O. ET AL: 'Ex vivo differentiation therapy as a method of leukemic cell purging in murine bone marrow expansion cultures' CANCER RESEARCH vol. 52, 1992, pages 6576 - 6582

## Description

### Field of the Invention

The present invention relates to methods of differentiating cells, pharmaceuticals related thereto, and methods of identifying compounds possibly useful in treating AIDS.

### Background of the Invention

Since the demonstration in 1987 that the small open reading frame within HIV-1 designated R encodes a 15 kd protein (Wong-Staal, F., *et al*., (1987) *AIDS Res. Hum. Retroviruses* **3**:33-39), relatively little regarding the function of the viral protein R (*vpr*) has been reported. WO 90/15875 disclosed a DNA sequence encoding a gene which can express the vpr gene product, the protein itself and on antibody which reacted with the protein. The vpr open reading frame is conserved within all genomes of HIV-1 and HIV-2 and within most, if not all, simian immunodeficiency virus (SIV) genomes. VPR is immunogenic in vivo in that a large subset of HIV⁺ individuals makes antibodies that can react with a bacterially produced vpr peptide (Wong-Staal, F., *et al*., (1987) *AIDS Res. Hum. Retroviruses* **3**:33-39).

The progression from HIV infection to AIDS is in large part determined by the effects of HIV on the cells that it infects, including CD4⁺ T lymphocytes and macrophages. On the other hand, cell activation, differentiation and proliferation are in turn thought to regulate HIV infection and replication in T cells and macrophages. Gallo, R.C. *et al*. (1984) *Science* **224**:500; Levy, J.A. *et al*., (1984) *Science* **225**:840; Zack, J.A. *et al*. (1988) *Science* **240**:1026; Griffin, G.E. *et al*., (1988) *Nature* **339**:70; Valentin, A. *et al*. (1991) *J. AIDS* **4**:751; Rich, E.A. *et al*., (1992) *J. Clin. Invest.* **89**:176; and Schuitemaker, H. *et al*. (1992) *J. Virol.* **66**:1354. Cell division *per se* may not be required since HIV and other lentiviruses can proliferate in nonproliferating, terminally differentiated macrophages and growth-arrested T lymphocytes. Rose, R.M. *et al*. (1986) *Am. Rev. Respir. Dis*. **143**:850; Salahuddin, S.Z. *et al*. (1986) *Blood* **68**:281; and Li, G. *et al*. (1993) *J. Virol*. **67**:3969. The ability of lentiviruses, including HIV, to replicate in nonproliferating cells, particularly in macrophages, is believed to be unique among retroviruses and it may be significant that several lentiviruses contain a vpr-like gene. Myers, G. *et al*. (1992) *AIDS Res. Hum. Retrovir.* **8**:373. HIV infection of myeloid cell lines can result in a more differentiated phenotype and increase the expression of factors such as NF-KB which are necessary for HIV replication. Roulston, A. *et al*. (1992) *J. Exp. Med*. **175**:751; and Chantal Petit, A.J. *et al*. (1987) *J. Clin. Invest.* **79**:1883.

The most evidence for the function of the vpr protein comes from several studies reporting the activities of HIV strains that have mutations in the vpr gene. It has been reported that mutations in the vpr gene results in a decrease in the replication and cytopathogenicity of HIV-1, HIV-2, and SIV in primary CD4⁺T lymphocytes and transformed T cell lines (Ogawa, K., *et al*., (1989) *J. Virol.* **63**:4110-4114; Shibata, R., *et al*. (1990a). *J. Med. Primatol*. **19**:217-225; Shibata, R., *et al*. (1990b) *J. Virol.* **64**:742-747 and Westervelt, P. *et al*. (1992) *J. Virol.* **66**:3925), although others have reported mutated vpr gene had no effect on replication (Dedera, D., *et al*. (1989) *Virol*. **63**:3205-3208). Interestingly HIV-2 mutated for vpr has been reported unable to infect primary monocyte/macrophages (Hattori, N., *et al*. (1990) *Proc. Natl. Acad. Sci. USA* **87**:8080-8084). Transactivation of the HIV long terminal repeat and heterologous promoters by HIV is increased about 3-fold in wild-type versus *vpr*-negative HIV-1, though the mechanism through which *vpr* may transactivate transcription is unknown and may be indirect (Cohen, E.A., *et al*., (1990b) *J. Acquir. Immune Defic. Syndr.* **3**:11-18). The relationship between the effects of vpr on promoter activity and viral infectivity is not clear. *Vpr* protein is incorporated into the viral particle, and this finding has led to the proposition that vpr functions early in infection, following virus penetration and uncoating, and that *vpr* may interact with cellular regulatory mechanisms important in the establishment of infection (Cohen, E.A., *et al*. 1990a *J. Virol*. **64**:3097-3099; Yu, X.F., *et al*. (1990) *J. Virol*. **64**:5688-5693.; and, Yuan, X., *et al*., (1990) *AIDS Res. Hum. Retroviruses* **6**:1265-1271).

The vpr gene of HIV-1 has been shown to induce cellular growth inhibition and differentiation in tumor lines of intermediate differentiation in vitro. Levy, D.N. *et al*. (1993) *Cell* **72**:541. Since vpr protein originates within viral particles, vpr may play a role in establishing productive infection.

### Summary of the Invention

The present invention relates to in vitro methods of inducing undifferentiated cells to differentiate. The present invention relates to a method of stimulating undifferentiated cells to differentiate which comprises the step of contacting cells in vitro with an amount of *vpr* protein sufficient to stimulate differentiation. According to some embodiments of the present invention, undifferentiated cells are contacted with *vpr* protein or a function fragment of *vpr* protein in order to induce the cells to differentiate. According to some embodiments of the present invention, a nucleic acid molecule that comprises a sequence which encodes *vpr* protein or a functional fragment of vpr protein is introduced into undifferentiated cells. Expression of the sequence that encodes the *vpr* protein or the functional fragment of *vpr* protein results in the production of the vpr protein or the functional fragment of vpr protein within the cell, causing the cell to differentiate. According to some embodiments of the present invention, the sequence which encodes *vpr* protein or a functional fragment thereof is operably linked to regulatory elements which are necessary for expression of the sequence in the cell. According to some embodiments of the present invention, the nucleic acid molecule is DNA.

The present invention relates to pharmaceutical compositions that comprise *vpr* protein and a pharmaceutically acceptable carrier. According to some embodiments of the present invention, the pharmaceutical composition comprises a functional fragment of vpr protein and a pharmaceutically acceptable carrier.

The present invention relates to pharmaceutical compositions that comprise a nucleic acid molecule that comprises a sequence which encodes vpr protein and a pharmaceutically acceptable carrier. According to some embodiments of the present invention, the pharmaceutical composition comprises a nucleic acid molecule that comprises a sequence which encodes a functional fragment of vpr protein and a pharmaceutically acceptable carrier. According to some embodiments of the present invention, the pharmaceutical composition comprises a nucleic acid molecule that comprises a sequence which encodes vpr protein or a functional fragment thereof that is operably linked to regulatory elements which are necessary for expression of the sequence in the cell. According to some embodiments of the present invention, a pharmaceutical composition comprises a nucleic acid molecule that is DNA.

The present invention relates to the use of an amount of vpr protein sufficient to stimulate hyperproliferating undifferentiated cells in the manufacture of a medicament to treat individuals diagnosed with or suspected of suffering from diseases characterized by hyperproliferating undifferentiated cells. According to some embodiments, the use of the present invention comprises the steps of administering to such individuals, an effective amount of vpr protein or a functional fragment of *vpr* protein. According to some embodiments of the present invention, the use of the present invention comprises the steps of administering to such individuals, an effective amount of a nucleic acid molecule that comprises a sequence which encodes vpr protein or a functional fragment of vpr protein. According to some embodiments of the present invention, the sequence that encodes vpr protein or a functional fragment of vpr protein is operably linked to regulatory elements which are necessary for expression of the sequence in cells. According to some embodiments of the present invention, the nucleic acid molecule is DNA. According to some embodiments of the present invention, the disease characterized by hyperproliferating undifferentiated cells is cancer or psoriasis.

The present invention relates to a method of identifying compounds which inhibit vpr from stimulating differentiation of undifferentiated cells which comprises the steps of first contacting, in the presence of a test compound, said cells with an amount of vpr protein sufficient to stimulate differentiation and then observing said cells to determine if cell differentiation occurs.

The present invention relates to vpr protein produced in eukaryotic cells. The vpr is preferably produced in human cells, CHO cells, insect cells or yeast cells.

### Description of Preferred Embodiments of the Invention

The present invention arises out of the discovery of activities of the HIV regulatory protein vpr (referred to herein as "vpr protein"). It has been discovered that HIV protein *vpr* induces undifferentiated cells to differentiate individuals infected with HIV as can antibodies that specifically bind to eukaryotic *vpr.* These activities and functions of *vpr* allow *vpr* to be useful in in vitro methods of altering cells including cancer cells and cells associated with autoimmune disease methods of identifying compounds that inhibit HIV infection and/or replication, and pharmaceutical compositions.

The invention relates to the *vpr's* ability to induce undifferentiated cells to differentiate. In some embodiments, *vpr* is used in a pharmaceutical composition to treat individuals suffering from diseases associated with hyperproliferating undifferentiated cells such as cancer or psoriasis. In some embodiments, *vpr* is used as a reagent to in vitro induce undifferentiated cells to differentiate. In some embodiments, undifferentiated tumor cells of specific cell type origin are induced to differentiate back to their prior cell type. The ability of *vpr* to stimulate differentiation is believed to assist the virus in replication by producing a desirable environment or conditions, particularly for production of viral particles. Accordingly, in one aspect of the invention, anti-HIV compounds may be identified by identifying compounds that inhibit the activity of *vpr* to induce differentiation in undifferentiated cells.

The present invention also relates to the use of functional fragments of *vpr* to in vitro induce differentiation of undifferentiated cells and to reagents and pharmaceutical compositions that comprise functional fragments of vpr and to uses of functional fragments of *vpr.* As used herein, the term "functional fragment of *vpr*" is meant to refer to a fragment of vpr which retains its ability to induce differentiation of undifferentiated cells. Functional fragment of *vpr* are at least about 5 amino acids in length derived from *vpr* and may comprise non-vpr amino acid sequences. One having ordinary skill in the art can readily determine whether a protein or peptide is a functional fragment of *vpr* by examining its sequence and testing its ability to differentiate undifferentiated cells without undue experimentation. Truncated versions of vpr may be prepared and tested using routine methods and readily available starting material. As used herein, the term "functional fragment" is also meant to refer to peptides, polypeptides, amino acid sequence linked by non-peptidal bonds, or proteins which comprise an amino acid sequence that is identical or substantially homologous to at least a portion of the vpr protein amino acid sequence and which are capable of inducing a hyperproliferating undifferentiated cell to differentiate. The term "substantially homologous" refers to an amino acid sequence that has conservative substitutions. One having ordinary skill in the art can produce functional fragments of vpr protein following the disclosure provided herein and well known techniques. The functional fragments thus identified may be used and formulated in place of full length vpr without undue experimentation.

Therapeutic aspects include use of *vpr,* a functional fragment of *vpr*, nucleic acid molecules encoding vpr or nucleic acid molecules encoding a functional fragment of *vpr* in the manufacture of pharmaceutical compositions useful to treat an individual suffering from diseases associated with hyperproliferating undifferentiated cells such as cancer or psoriasis.

One aspect of the present invention is to use vpr, a functional fragment of vpr, nucleic acid molecules encoding vpr or nucleic acid molecules encoding a functional fragment of *vpr* in the manufacture of a pharmaceutical composition to combat diseases that are characterized by the hyperproliferation of undifferentiated cells such as cancer or psoriasis. According to the invention, pharmaceutical compositions are provided which comprise either *vpr* protein or a functional fragment thereof or a nucleic acid molecule which comprises a DNA or RNA sequence that encodes vpr protein or a functional fragment thereof.

One aspect of the present invention relates to pharmaceutical compositions that comprise HIV protein vpr or a functional fragment thereof and a pharmaceutically acceptable carrier or diluent. Pharmaceutical compositions comprising *vpr* protein or a functional fragment thereof are useful for treating an individual having a pathology or condition characterized by hyperproliferating undifferentiated cells. As described herein, pharmaceutical compositions useful for treating diseases characterized by hyperproliferating undifferentiated cells may include *vpr* protein or a functional fragment thereof since vpr protein or a functional fragment thereof are by definition agents which induce undifferentiated cells to differentiate. Pharmaceutical compositions of the present invention are particularly useful for treating cancer characterized by solid tumors. The ability to stimulate hyperproliferating undifferentiated cells to differentiate provides a means to disrupt the hyperproliferation of the cells. In diseases such as cancer and psoriasis which are characterized by the hyperproliferation of undifferentiated cells, the pharmaceutical composition is useful to stimulate the undifferentiated cells to differentiate. When hyperproliferating undifferentiated cells are induced to differentiate, they cease proliferating and eventually die.

Accordingly, another aspect of the present invention is the use of an amount of vpr protein sufficient to stimulate differentiation of hyperproliferating undifferentiated cells in the manufacture of a medicament to treat an individual suffering from a disease associated with hyperproliferating undifferentiated cells.

*Vpr* may be produced by routine means using readily available starting materials as described above. The nucleic acid sequence encoding vpr as well as the amino acid sequence of the protein are well known. The entire HIV genome is published. The long terminal repeat sequences are reported in Stacich, B. *et al*., (1985) *Science* **227**:538-540. Complete nucleotide sequences are reported in Ratner, L. *et al*., (1985) *Science* **313**:277-284 and Ratner, L. *et al*., (1987) *AIDS Res. Hum. Retroviruses* **3**:57-69. The DNA sequence of HIV-1/3B is published in Fisher, A., 1985 *Nature* **316**:262,. The HIV-1 HXB2 strain nucleotide sequence is available on line from Genbank accession number K03455. The HIV DNA sequence is published in Reiz, M.S., 1992 *AIDS Res.* Human *Retro.* **8**:1549. The sequence is accessible from Genbank No.: M17449. Each of these references including the publicly available sequence information are incorporated herein by reference.

DNA molecules that encode vpr are readily available to the public. Plasmid pNL-43 which contains a DNA sequence encoding HIV-1 strain MN including the vpr protein and plasmid pHXB2 which contains a DNA sequence encoding HIV strain HIV-1/3B are both available from AIDS Research Reference and Reagent Program (ARRRP), Division of AIDS, NIAID, NIH, Bethesda, MD.

Provision of a suitable DNA sequence encoding the desired protein permits the production of the protein using recombinant techniques now known in the art. The coding sequence can be obtained by retrieving the DNA sequence from the publicly available plasmids which comprise DNA encoding *vpr* protein. The DNA sequence may also be obtained from other sources of HIV DNA or can be prepared chemically using a synthesized nucleotide sequence. When the coding DNA is prepared synthetically, advantage can be taken of known codon preferences of the intended host where the DNA is to be expressed.

One having ordinary skill in the art can, using well known techniques, obtain a DNA molecule encoding the *vpr* protein and insert that DNA molecule into a commercially available expression vector for use in well known expression systems. For example, the commercially available plasmid pSE420 (Invitrogen, San Diego, CA) may be used for production in *E. coli.* The commercially available plasmid pYES2 (Invitrogen, San Diego, CA) may be used for production in *S. cerevisiae* strains of yeast. The commercially available MaxBac^{™} (Invitrogen, San Diego, CA) complete baculovirus expression system may be used for production in insect cells. The commercially available plasmid pcDNA I (Invitrogen, San Diego, CA) may be used for production in may be used for production in mammalian cells such as Chinese Hamster Ovary cells.

One having ordinary skill in the art can use these commercial expression vectors systems or others to produce vpr protein using routine techniques and readily available starting materials.

One having ordinary skill in the art may use other commercially available expression vectors and systems or produce vectors using well known methods and readily available starting materials. Expression systems containing the requisite control sequences, such as promoters and polyadenylation signals, and preferably enhancers, are readily available and known in the art for a variety of hosts. *See e.g.,* Sambrook et al., *Molecular Cloning a Laboratory Manual,* Second Ed. Cold Spring Harbor Press (1989). Thus, the desired proteins can be prepared in both prokaryotic and eukaryotic systems, resulting in a spectrum of processed forms of the protein.

The most commonly used prokaryotic system remains *E. coli,* although other systems such as *B. subtilis* and *Pseudomonas* are also useful. Suitable control sequences for prokaryotic systems include both constitutive and inducible promoters including the *lac* promoter, the trp promoter, hybrid promoters such as tac promoter, the lambda phage P1 promoter. In general, foreign proteins may be produced in these hosts either as fusion or mature proteins. When the desired sequences are produced as mature proteins, the sequence produced may be preceded by a methionine which is not necessarily efficiently removed. Accordingly, the peptides and proteins claimed herein may be preceded by an N-terminal Met when produced in bacteria. Moreover, constructs may be made wherein the coding sequence for the peptide is preceded by an operable signal peptide which results in the secretion of the protein. When produced in prokaryotic hosts in this matter, the signal sequence is removed upon secretion.

A wide variety of eukaryotic hosts are also now available for production of recombinant foreign proteins. As in bacteria, eukaryotic hosts may be transformed with expression systems which produce the desired protein directly, but more commonly signal sequences are provided to effect the secretion of the protein. Eukaryotic systems have the additional advantage that they are able to process introns which may occur in the genomic sequences encoding proteins of higher organisms. Eukaryotic systems also provide a variety of processing mechanisms which result in, for example, glycosylation, carboxy-terminal amidation, oxidation or derivatization of certain amino acid residues, conformational control, and so forth.

Commonly used eukaryotic systems include, but is not limited to, yeast, fungal cells, insect cells, mammalian cells, avian cells, and cells of higher plants. Suitable promoters are available which are compatible and operable for use in each of these host types as well as are termination sequences and enhancers, as e.g. the baculovirus polyhedron promoter. As above, promoters can be either constitutive or inducible. For example, in mammalian systems, the mouse metallothionene promoter can be induced by the addition of heavy metal ions.

The particulars for the construction of expression systems suitable for desired hosts are known to those in the art. For recombinant production of the protein, the DNA encoding it is suitably ligated into the expression vector of choice and then used to transform the compatible host which is then cultured and maintained under conditions wherein expression of the foreign gene takes place. The protein of the present invention thus produced is recovered from the culture, either by lysing the cells or from the culture medium as appropriate and known to those in the art.

One having ordinary skill in the art can, using well known techniques, isolate the vpr protein produced using such expression systems.

In addition to producing these proteins by recombinant techniques, automated amino acid synthesizers may also be employed to produce vpr protein. It should be further noted that if the proteins herein are made synthetically, substitution by amino acids which are not encoded by the gene may also be made. Alternative residues include, for example, the ω amino acids of the formula H₂N(CH₂)ₙCOOH wherein n is 2-6. These are neutral, nonpolar amino acids, as are sarcosine (Sar), t-butylalanine (t-BuAla), t-butylglycine (t-BuGly), N-methyl isoleucine (N-MeIle), and norleucine (Nleu). Phenylglycine, for example, can be substituted for Trp, Tyr or Phe, an aromatic neutral amino acid; citrulline (Cit) and methionine sulfoxide (MSO) are polar but neutral, cyclohexyl alanine (Cha) is neutral and nonpolar, cysteic acid (Cya) is acidic, and ornithine (Orn) is basic. The conformation conferring properties of the proline residues may be obtained if one or more of these is substituted by hydroxyproline (Hyp).

The pharmaceutical composition comprising vpr protein and a pharmaceutically acceptable carrier or diluent may be formulated by one having ordinary skill in the art with compositions selected depending upon the chosen mode of administration. Suitable pharmaceutical carriers are described in the most recent edition of *Remington's Pharmaceutical Sciences,* A. Osol, a standard reference text in this field.

For parenteral administration, the vpr protein can be, for example, formulated as a solution, suspension, emulsion or lyophilized powder in association with a pharmaceutically acceptable parenteral vehicle. Examples of such vehicles are water, saline, Ringer's solution, dextrose solution, and 5% human serum albumin. Liposomes and nonaqueous vehicles such as fixed oils may also be used. The vehicle or lyophilized powder may contain additives that maintain isotonicity (e.g., sodium chloride, mannitol) and chemical stability (e.g., buffers and preservatives). The formulation is sterilized by commonly used techniques. For example, a parenteral composition suitable for administration by injection is prepared by dissolving 1.5% by weight of active ingredient in 0.9% sodium chloride solution.

The pharmaceutical compositions according to the present invention may be administered as a single doses or in multiple doses. The pharmaceutical compositions of the present invention may be administered either as individual therapeutic agents or in combination with other therapeutic agents. The treatments of the present invention may be combined with conventional therapies, which may be administered sequentially or simultaneously.

The pharmaceutical compositions comprising vpr protein, or fragments or derivatives may be administered by any means that enables the active agent to reach the agent's site of action in the body of a mammal. Because proteins are subject to being digested when administered orally, parenteral administration, i.e., intravenous, subcutaneous, intramuscular, would ordinarily be used to optimize absorption. In addition, the pharmaceutical compositions of the present invention may be injected at a site at or near hyperproliferative growth. For example, administration may be by direct injection into a solid tumor mass or in the tissue directly adjacent thereto. If the individual to be treated is suffering from psoriasis, the vpr protein may be formulated with a pharmaceutically acceptable topical carrier and the formulation may be administered topically as a creme, lotion or ointment for example.

The dosage administered varies depending upon factors such as: pharmacodynamic characteristics; its mode and route of administration; age, health, and weight of the recipient; nature and extent of symptoms; kind of concurrent treatment; and frequency of treatment. Usually, a daily dosage of *vpr* protein can be about 1µg to 100 milligrams per kilogram of body weight. Ordinarily 0.5 to 50, and preferably 1 to 10 milligrams per kilogram per day given in divided doses 1 to 6 times a day or in sustained release form is effective to obtain desired results.

Another aspect of the present invention relates to pharmaceutical compositions that comprise a nucleic acid molecule that encodes vpr and a pharmaceutically acceptable carrier or diluent. According to the present invention, genetic material that encodes vpr protein is delivered to an individual in an expressible form. The genetic material, DNA or RNA, is taken up by the cells of the individual and expressed. The *vpr* protein that is thereby produced can stimulate hyperproliferating undifferentiated cells to differentiate. Thus, pharmaceutical compositions comprising genetic material that encodes vpr protein are useful in the same manner as pharmaceutical compositions comprising vpr protein: for treating an individual having a pathology or condition characterized by hyperproliferating undifferentiated cells. Pharmaceutical compositions of the present invention are particularly useful for treating cancer characterized by solid tumors.

Thus, a further aspect of the present invention relates to an amount of nucleic acid that comprises a nucleotide sequence that encodes *vpr* protein operably linked to regulatory elements necessary for expression in the manufacture of a medicament to treat an individual suffering from a disease associated with hyperproliferating undifferentiated cells.

Nucleotide sequences that encode *vpr* protein operably linked to regulatory elements necessary for expression in the individual's cell may be delivered as pharmaceutical compositions using gene therapy strategies which include, but are not limited to, either viral vectors such as adenovirus or retrovirus vectors or direct nucleic acid transfer. Methods of delivery nucleic acids encoding proteins of interest using viral vectors are widely reported. A recombinant viral vector such as a retrovirus vector or adenovirus vector is prepared using routine methods and starting materials. The recombinant viral vector comprises a nucleotide sequence that encodes vpr. Such a vector is combined with a pharmaceutically acceptable carrier or diluent. The resulting pharmaceutical preparation may be administered to an individual. Once an individual is infected with the viral vector, vpr protein is produced in the infected cells.

Alternatively, a molecule which comprises a nucleotide sequence that encodes vpr can be administered as a pharmaceutical composition without the use of infectious vectors. The nucleic acid molecule may be DNA or RNA, preferably DNA. The DNA molecule may be linear or circular, it is preferably a plasmid. The nucleic acid molecule is combined with a pharmaceutically acceptable carrier or diluent.

According to the invention, the pharmaceutical composition comprising a nucleic acid sequence that encodes vpr protein may be administered directly into the individual or delivered ex vivo into removed cells of the individual which are reimplanted after administration. By either route, the genetic material is introduced into cells which are present in the body of the individual. Preferred routes of administration include intramuscular, intraperitoneal, intradermal and subcutaneous injection. Alternatively, the pharmaceutical composition may be introduced by various means into cells that are removed from the individual. Such means include, for example, transfection, electroporation and microprojectile bombardment. After the nucleic acid molecule is taken up by the cells, they are reimplanted into the individual.

The pharmaceutical compositions according to this aspect of the present invention comprise about 0.1 to about 1000 micrograms of DNA. In some preferred embodiments, the pharmaceutical compositions contain about 1 to about 500 micrograms of DNA. In some preferred embodiments, the pharmaceutical compositions contain about 25 to about 250 micrograms of DNA. Most preferably, the pharmaceutical compositions contain about 100 micrograms DNA.

The pharmaceutical compositions according to this aspect of the present invention are formulated according to the mode of administration to be used. One having ordinary skill in the art can readily formulate a nucleic acid molecule that encodes vpr. In cases where intramuscular injection is the chosen mode of administration, an isotonic formulation is used. Generally, additives for isotonicity can include sodium chloride, dextrose, mannitol, sorbitol and lactose. Isotonic solutions such as phosphate buffered saline may be used. Stabilizers include gelatin and albumin.

Another aspect of the present invention relates to a method of stimulating undifferentiated cells to differentiate which comprises the step of contacting said cells in vitro with an amount of *vpr* protein or a nucleic acid molecule that encodes *vpr* sufficient to stimulate differentiation.

Another aspect of the present invention relates to a method of identifying compounds which inhibit vpr from stimulating differentiation of undifferentiated cells which comprises the steps of first contacting, in the presence of a test compound, said cells with an amount of vpr protein sufficient to stimulate differentiation and then observing said cells to determine if cell differentiation occurs. It is believed that *vpr*'s ability to stimulate undifferentiated cells to differentiate is important for the efficient production of viral particles during HIV infection. Identifying compounds which interfere with *vpr*'s stimulation of cell differentiation provides a drug target for combatting the virus.

According to this aspect of the invention, compounds are identified which modulate *vpr* stimulation of differentiation of undifferentiated cells. An assay is provided which compares differentiation stimulation by vpr in the presence or absence of test compounds. Using this assay, compounds can be identified which modulate vpr stimulatory activity. In particular, compounds can be identified which inhibit vpr stimulatory action. Such compounds may be useful as anti-HIV therapeutics.

The method of the present invention comprises the step of contacting undifferentiated cells with *vpr* in the presence of a test compound. The cells can then be observed to determine if the *vpr* induces differentiation. A control may be provided in which *vpr* is contacted with cells in the absence of test compound. A further control may be provided in which test compound is contacted with cells in the absence of *vpr*. If the cells contacted with vpr in the presence of test compound do not differentiate, then anti-*vpr* activity is indicated for the test compound. This can be confirmed if cells contacted with vpr in the absence of test compound differentiate and the cells contacted with test compound in the absence of vpr do not differentiate.

The assay may be performed using many different types of undifferentiated cells and delivery of vpr through a variety of means. Additionally, functional fragments of vpr may be used in place of *vpr*. One having ordinary skill in the art, following the teachings of the Specification, can readily appreciate the several ways to practice this aspect of the present invention.

Undifferentiated cells include stem cells except human embryonic stem cells in the context of the present invention, and transformed cells such as cultured tumor cells. It is preferred that the cell type chosen is one in which the differentiated form is readily distinguishable from undifferentiated cells. In some embodiments of the invention, the preferred cell types are those of the solid muscle tumor alveolar rhabdomyosarcoma such as the cell lines RD, TE671 and D17. MG63 and HOS-TE86, which are examples of osteosarcoma cell lines, may also be used. KG-1, THP-1, U937, HL60, and PLB973 cell lines are examples of myeloid lineage cells which may be used in the assay. Other cell lines that may be used in the assay include human glioblastoma cell line U-138MG, the human glioblastoma/astrocytoma cell line U373MG and the human glioblastoma/astrocytoma cell line U87-MG.

Test compound is provided, preferably in solution. Serial dilutions of test compounds may be used in a series of assays. Test compound may be added at concentrations from 0.01µM to 1M. A preferred range of final concentrations of a test compound is from 10µM to 100µM. One test compound that is effective to inhibit *vpr*'s activity is an antibody that specifically binds to vpr and prevents it from inducing differentiation of undifferentiated cells.

*Vpr* may be delivered by a variety of means. In some embodiments of the invention, it is combined with cells as a protein. The *vpr* protein may be added directly to cell culture medium. *Vpr* protein may be produced from widely available starting materials using well known techniques, such as described above. A preferred concentration range of the *vpr* used is about 1µg/ml to 1 mg/ml.

Alternatively, *vpr* may be contacted with undifferentiated cells by introducing into the cell a nucleic acid molecule which comprises a nucleic acid sequence encoding *vpr.* In such embodiments, the nucleic acid sequence may be introduced as part of an HIV particle, part of a recombinant infectious expression system particle or part of an expression vector such as a plasmid. Additionally linear DNA or RNA may also be introduced into the cell in an expressible form. One having ordinary skill in the art can construct any number of expression vectors or other nucleic molecules designed to produce vpr in cultured cells. Such an expression system may include a vector system to introduce the genetic material or the nucleic acid molecule may be introduced by other standard techniques such as transfection, electroporation or microprojectile bombardment.

Those having ordinary skill in the art can distinguish undifferentiated cells from differentiated cells routinely. Methods of distinguishing differentiated cells from undifferentiated cells include observing morphological, metabolic and biochemical differences between cell stages. For example, differences in size, shape and over all appearance are often profound when comparing an undifferentiated cell from a corresponding differentiated cell. Likewise, differentiation of cells results in changes in the proteins being produced by the cell.

For example, differentiated alveolar rhabdomyosarcoma cells produce high levels of myosin, a muscle protein, relative to the level of myosin produced by undifferentiated alveolar rhabdomyosarcoma cells. When undifferentiated alveolar cells are induced to differentiate, the increase in the presence of myosin may be detected using routine techniques. The means to detect the presence of a protein product are routine and include enzyme assays and ELISA assays. One having ordinary skill in the art can detect the presence or absence of a protein using well known methods.

Specifically, the initial set of cell lines which were studied included RD, TE671 and D17 as representatives of rhabdomyosarcoma (muscle) cell lines. Differentiation markers for these cells include skeletal alpha-actin, myosin, muscle specific creatine kinase, and troponin 1.

The effects of *vpr* on expression of the differentiated osteoblast phenotype in the osteosarcoma cell lines MG63 and HOS-TE86 can be observed using non-specific markers of alteration in cell function such as morphology and cell proliferation as well as the expression of osteoblastic markers. The osteoblast markers include the expression of mRNA's for osteocalcin, alkaline phosphatase and type I (aI) collagen (by Northern analysis) and the synthesis of osteocalcin (by radioimmunoassay) and alkaline phosphatase (colorimetric assay). The specificity of effects of test compounds on vpr are also compared to compounds effects on other established osteoblast differentiating agents such as retinoid acid and 1,25 dihydroxyvitamin D₃.

Differentiation analysis in cell lines KG-1, THP-1, U937, HL60, and PLB973 cell lines, which are of myeloid lineage, include increases in plastic adherence, increased phagocytosis of latex beads, positive staining for alpha-naphthyl acetate esterase and loss of expression of elastase and cathepsin G, for example. Additionally differentiation of myeloid cell lines can be correlated with changes in specific oncogene expression such as decreases in c-*myc* transcription.

During differentiation of glioblastoma cell lines, such as the human glioblastoma cell line U-138MG, the human glioblastoma/astrocytoma cell line U373MG and the human glioblastoma/astrocytoma cell line U87-MG, there is a decrease in cell proliferation, increases in ornithine decarboxylase, increases in GFAP, transient increases in fos, increases in specific collagen, increases in the cytoplasmic to nuclear rations, pseudopod extension, neurite outgrowth, bipolarity and activated cytoskeletal activity. Additionally, during differentiation of astrocytes increases in fibronectin expression have been reported.

While the portions of the disclosure herein which relate to therapeutic compositions primarily relate to therapeutics useful for treating humans, the compositions of the present invention can be applied to veterinary medical uses as well.

### Examples

### Example 1

### Summary

The vpr gene of HIV-1 is sufficient for the differentiation of the human rhabdomyosarcoma cell line TE671, a cell lines from rhabdomyosarcomas, which are tumors of muscle origin and which can be induced to differentiate *in vitro*. Differentiated cells are characterized by great enlargement, altered morphology, lack of replication, and high level expression of the muscle-specific protein myosin. Morphological differentiation and inhibition of proliferation of two other transformed cell lines has also been observed. *vpr*-transfected cells remain fully viable in culture for extended periods.

The development of mature skeletal muscle cells entails an ordered process of cellular differentiation from muscle-committed mycocytes (presumptive myoblasts), to postmitotic myoblasts, to mature multinucleated myotubes possessing a functional muscle-contractile apparatus. Embryonal rhabdomyosarcoma is a cancer of cells resembling presumptive myoblasts and may originate from muscle satellite cells (Bruni, 1979). Rhabdomyosarcoma cell lines have been used in studies of muscle differentiation and tumorigenesis, and they can be induced to differentiate from a rapidly dividing population of cells (myoblast-like) that express low amounts of a few mature-muscle proteins to postmitotic, greatly enlarged and elongated, multinucleated (myotube-like) cells that express high amounts of mature-muscle-specific proteins and a functional muscle-contractile apparatus (Aguanno, S., *et al*., (1990) *Cancer Res.* **50**:3377-3382; Hiti, A.L., *et al*., (1989) *Hol. Cell. Biol*. **9**:4722-4730; Siegel, H.N., and Lukas, R.J. (1988) *Dev. Brain Res*. **44**:269-280; and Stratton, M.R., *et al*., (1989) *Carcinogenesis* **10**:899-905.)

HIV expression in a human muscle cell tumor line leads to inhibition of proliferation and activation of the suppressed endogenous cell differentiation program. The vpr gene of HIV-1 is sufficient for the observed effects and necessary for differentiation of essentially all cells. These results establish HIV-1 vpr as a regulatory protein capable of profound regulation of cell functions, including cell proliferation and differentiation.

### Experimental Procedures

### Cell Lines and Cultivation

The human embryonal rhabdomyosarcoma TE671 line (ATCC HTB 139) and the canine osteosarcoma D17 line (ATCC CLL 183) were obtained from the American Type Culture Collection, Rockville, Maryland. TE671 was originally classified as a medulloblastoma line. RD cells were provided by Dr. A. Srinivasan. All cells were grown in Dulbecco's modified Eagle's medium supplemented with 10% fetal calf serum, penicillin-streptomycin, and sodium pyruvate and maintained in a 5%-6% CO₂ atmosphere at 37°C.

### Construction of the TE671Ψ Cell Line

TE671 cells were infected with replication-defective murine retrovirus containing the human CD4 retroviral expression vector T4-pMV7 as described in Weiner, D.B., *et al*., (1991). *Pathobiology* **59**:361-371. Clonal populations were analyzed for CD4 expression by flow cytometry as described in Weiner, D.B., *et al*., (1989) *Oncogene* **4**:1175-1183. Briefly, cells were incubated with either Leu-3a, a murine monoclonal antibody specific for the human CD4 cell surface molecule, or Upc-21, an irrelevant isotype-matched murine monoclonal antibody. Secondary antibody was a fluorescein-labeled goat anti-mouse antibody. A stable CD4⁺ clone was selected for further analysis and designed TE671Ψ

### Plasmids and Cloning Strategies

The HIV-1 genomic clone pNL43 was obtained through the National Institutes of Health (NIH) AIDS Research and Reference Reagent Program, Division of AIDS, National Institute of Allergy and Infectious Diseases, NIH, (Adachi, A., *et al*. (1986) *J. Virol.* **59**:284-291), and was used as the starting material for most of the genetic constructs used in this study. The pNL43 plasmid consists of HIV-1 proviral DNA plus 3 kb of host sequence from the site of integration cloned into pUC18.

### Construction of pNLpuro

To simplify further cloning steps, the *Stu*l site within the non-HIV 5' flanking human DNA of pNL43 was destroyed by partial digestion with *Stu*l followed by digestion of the free ends with *Escherichia coli* polymerase. The linear plasmid was filled, then self-ligated, leaving a unique *Stu*l site within the HIV genome. This plasmid, pNLΔstu, was then digested with the blunting enzymes *Stu*l and *Bsa*Bl, which eliminated a large section of the coding sequence for gp120. The SV40 promoter and puromycin resistance coding region (puromycin acetyltransferase) were isolated from pBABE-puro (Morganstern, J.P., and Land, H. (1990). *Nucl. Acids Res.* **18**:3587-3596; kindly provided by Dr. Hartmut Land of the Imperial Cancer Research Fund) using *Eco*Rl and *Cla*l This fragment was blunted, then cloned into the *Stu*l-*Bsa*Bl-digested pNLΔstu. A clone was selected with the SV40-puro fragment in the correct orientation so that the 3' long terminal repeat of HIV could provide poly(A) functions for the puromycin acetyltransferase message. This plasmid was designated pNLpuro.

### HIV-1 Regulatory Genes

DNA encoding HIV-1 vpr protein was amplified via PCR from the HIV-1 genomic plasmid pNL43. PCR was performed under conditions that yield DNA amplification with highly fidelity (Ling, L.L., *et al*., (1991) *PCR Meth. Appl.* **1**:63-69). Three PCR primers were used to amplify the gene. One primer, called a universal start/cloning primer (USP), encodes appropriate restriction sites for cloning the PCR product into a vector, a consensus sequence determined by Kozak, M. (1986) *Cell* **44**:283-292, to promote strong initiation of translation, and an ATG start site. This primer was used in the amplification of the gene. The USP was placed in a PCR with a second short primer consisting of a reverse complement of the 3' end of the USP plus approximately 15 bp of the 5' end of the vpr gene. The double-stranded product of this PCR was used as a 5' primer in a second reaction with an appropriate 3' primer specific for the gene of interest. The 3' primer introduced restriction sites for cloning the PCR product. The sequences of the primers are as follows:
USP:ggcggctcgaggatccgccgccaccatg (SEQ ID NO:1)*.*

*vpr* primer: 5' linker primer, complementary to the USP and the *vpr* open reading frame 5' end: ggggcttgttccatggtggc (SEQ ID NO:2)*.*

*vpr* primer: 3' cloning primer, complementary to the 3' end of the *vpr* open reading frame plus the *Bam*Hl cloning site: ccgcggatcctaggatctactggc (SEQ ID NO:3).

The resulting PCR product was cloned into the retroviral vector pBABE-puro. The vector pbabe-puro, which is used as a starting material to produce many of the below listed constructs, was originally constructed and reported by Morgenstern, J.P. and H. Land, 1990 *Nucl. Acids Res.* **18**(12):3587-3596, which is incorporated herein by reference. The pBabe-puro plasmid is particularly useful for expression of exogenous genes in mammalian cells. DNA sequences to be expressed are inserted at cloning sites under the control of the Moloney murine leukemia virus (Mo MuLV) long terminal repeat (LTR) promoter. The plasmid contains the selectable marker for puromycin resistance. The resulting plasmid is designated pBabe-puro+vpr.

HIV regulatory genes *nef, vpu* and *vif* were amplified via PCR from the HIV-1 genomic plasmid pNL43. Primers designed to amplify the remaining regulatory genes (*vif, vpu, nef*) were constructed by the same design principle as employed in the amplification of *vpr*. Each gene was then cloned into pBabe-puro.

### Cloning Strategy for Deletion of the vpr Gene from the HIV Genome

A region from just upstream of the unique *Pfl*MI site to just after the *vif* termination codon was amplified via PCR using primers that introduced a nonconservative amino acid change (Glu → Val) at amino acid 22 of *vpr,* a stop codon in the vpr reading frame immediately after amino acid 22, and an *Eco*Rl site immediately following the new stop codon. This PCR fragment was substituted for the *Pfl*Ml-*Eco*Rl fragment of pNLpuro or pNL43. This substitution resulted in the deletion of 122 nt of the open reading frame of *vpr*, thus eliminating the possibility of reversion. The resulting plasmids, pNLpuroΔvpr and pNLΔvpr, encode the first 21 natural amino acids of vpr plus a valine plus all other remaining HIV-1 genes and splice junctions in their native form.

### HIV-1 env-rev Plasmid

The region encoding the two exons of rev and the *vpu* and env open reading frames of HIV-1 HXB2 was amplified via PCR and cloned into the expression vector pCDNAl/neo (Invitrogen). Expression of rev and env proteins was demonstrated by Western blot analysis and by the ability of cells transfected with this construct to fuse with CD4⁺ cell lines.

### tat

HIV-1 tat expression plasmid pCV1 was obtained through the Aids Research and Reference Reagent Program. A region from the vector pBABE-hygro (Morganstern, J.P., and Land, H. (1990). *Nucl. Acids Res.* **18**:3587-3596) expressing hygromycin resistance was subcloned into this plasmid to make pCV1-hygro. Alternatively, pCV1 was cotransfected with pBABE-puro at a ratio of 100:1. Identical results were obtained with both methods.

### Determination of Regulatory Gene Expression by Reverse Transcription PCR

TE671 cells (0.5 x 10⁶ to 1.0 x 10⁶) were transfected using DOTAP (Boehringer Mannheim) with expression vectors encoding individual regulatory genes. Forty-eight hours later cells were lysed *in situ* using RNA_{20L} B (Biotecx Laboratories, Inc.), and total cellular RNA was prepared according to standard methodology. cDNA was prepared by reverse transcription using random 6-mer primers and Moloney murine leukemia virus reverse transcriptase. An aliquot of cDNA was used as a template in PCR amplification. As a control for possible genomic DNA contamination, aliquots of RNA not subject to reverse transcription were used as templates in PCR.

### Analysis of HIV-1 p24^{gag} Antigen Production

For analysis of the infectivity of TE671 and TE671Ψ, cells were grown to 80% confluence in tissue culture flasks, then incubated with filtered (0.2 µm pore size) supernatants from HUT-78 cells chronically infected with HIV1 (strain RF). One day later cells were washed once with phosphate-buffered saline containing trypsin (2.5 mg./ml), then twice with culture medium to remove residual virus used to infect the cells. Supernatants were collected at 24 hr intervals with the first collection occurring immediately after the wash step. Detection of p24^{gag} antigen was performed using an HIV1 p24 antigen assay kit (Coulter Immunology, Coulter Corporation) as per the manufacturer's instructions. This method employs an antigen-capture enzyme-linked immunosorbent assay. Wells were analyzed for absorbance at 450 nm on a Dynatech MR5000 enzyme-linked immunosorbent assay reader.

### Transfections for Differentiation Studies

For differentiation experiments TE671 cells were transfected either by electroporation or with the lipid-mediated method using DOTAP. Though DOTAP produced more efficient transfections than electroporation, identical results were obtained with both methods with respect to differentiation. RD and D17 cells were transfected using DOTAP. Briefly, electroporation was performed with a Bio-Rad GenePulser and Pulse Controller on 2 x 10⁴ to 5 x 10⁴ cells harvested in log phase growth. DOTAP transfection was performed as per the manufacturer's instructions in tissue culture flasks on 0.5 x 10⁴ to 1 x 10⁴ in log phase growth. In either case selection medium was added 48-60 hr after transfection, and cells were maintained in selection for the duration of the experiments. Cells transfected with plasmids containing the puromycin resistance gene (puromycin acetyltransferase) were selected in 1 µg/ml puromycin. Neomycin selection was in mg/ml G418.

### Anti-Myosin Photomicrographic Immunofluorescence Assay

TE671 cells were transfected with the *vpr* expression vector, and 48 hr later the cells were trypsinized, transferred to glass slides, and selected with puromycin for 5 days prior to staining. Rapidly proliferating untransfected TE671 cells were grown on glass slides for 2-4 days before staining. Permeabilization and fixation were performed with 100% methanol at -20°C for 10 min. The remaining steps were performed in PHEM buffer, which consists of 25mM HEPES, 60 mM PIPES, 10mM EGTA, and 2mM MgCl₂ (pH 6.9). The fixed cells were washed three times with PHEM in between each step. Cells were first blocked with 5% normal goat serum to reduce nonspecific staining. Murine monoclonal antibody MY-32, which is specific for the myosin heavy chain of fast-twitch (type II) skeletal muscle (Sigma number M-4276), was then incubated with the specimens. As a negative control, an isotype-matched antibody (SIM.4 anti-CD4, obtained through the AIDS Research and Reference Reagent Program from Dr. James Hildreth) was used as primary antibody on some cells. Rhodamine-conjugated goat anti-mouse immunoglobulin G (TAGO) was used as secondary antibody, or alternatively a peroxidase-conjugated secondary antibody was used (Boehringer Mannheim). The cells were washed with PHEM, then examined under a fluorescence microscope and photographed.

### Infection Assay

CD4⁺ TE671Ψ or CD4⁻ TE671 cells were plated into tissue culture at very low confluence (<5%). One day later, supernatant and infected cells were added from HIV-1 (strains RF or MN)-infected CD4⁺ HUT-78 T lymphoma cells. The following day the infected cells were washed from the culture, and fresh medium was added to the cells. Cells were examined for differentiation beginning on the second day after infection.

### Results

### Differentiation of TE671 Following Transfection with Genomic Construct pNLpuro

A drug-selectable env deletion mutant HIV-1 genomic plasmid, pNLpuro, based on the HIV-1 infectious molecular clone pNL43 was construct. pNLpuro was transfected into the human rhabdomyosarcoma cell line TE671 and selected for stable transfectants. TE671 cells normally grow as small mononuclear round or polygonal fibroblast-like adherent cells about 3-7 µm in length and developed long, sometimes branched, processes. Some cells became large, flat, and irregularly shaped. Differentiating cells often became bi- or multinucleated, though cells with long processes that resembled myotubes lacked the linear arrangement of many nuclei that is found in true myotubes. The large cells remained fully viable for several weeks.

The differentiation of TE671 cells via chemical agents is a well-described phenomenon, as this cell line has been used as a model for skeletal muscle differentiation. Some agents, including protein kinase C-activating phorbol esters, as well as serum depleted medium, will induce TE671 cells to undergo alterations in both morphology and growth characteristics that parallel in many aspects the differentiation of myoblasts into myotubes. Phorbol myristate acetate-stimulated TE671 cells increase in overall size and length, are often multinucleated, and display slowed proliferation. On the other hand, no morphologically differentiated pNLpuro-transfected cells were observed to divide when followed for up to 10 days.

### Determination of HIV-1 Elements Sufficient to Differentiation

To define the viral gene(s) responsible for induction of cell differentiation in the rhabdomyosarcoma cell line, individual HIV-1 regulatory genes were examined, as some of their protein products have been reported to influence cellular events. A tat expression vector (Arya, S.K., *et al*., (1985) *Science* **229**:69-73) was obtained and modified to permit selection of stably transfected cells. The *env-rev* region of HIV-1 was amplified by the polymerase chain reaction (PCR) from an HIV-1 molecular clone and subcloned directly into the pCDNAl/neo expression vector. To clone the remaining regulatory genes into expression vectors, the single open reading frame of each gene was amplified using PCR. During the PCR amplification a consensus ribosome initiation sequence was introduced immediately upstream of each start codon. The genes encoding the regulatory proteins *nef, vpr, vif*, and *vpu* were amplified by this method and subcloned into the pBABE-puro expression vector.

Each plasmid was transfected into TE671 cells and selected on the appropriate antibiotic. Expression of rev was demonstrated indirectly by showing expression of envelope protein (*env*) in Western blot and cell fusion assays. Since expression of env is dependent on a critical threshold level of rev expression, it can be deduced that physiologically relevant levels of *rev* were produced. The *vpr* protein expressed from the *vpr* vector showed a single 15 kd species by Western blot analysis. Cellular expression of *tat, vif, vpr, vpu,* and *nef* was demonstrated by reverse transcription PCR analysis. PCR products were run on 2% agarose gels and stained with ethidium bromide for photography. As a control against possible DNA carryover in the RNA preparations. RNA that was not subjected to reverse transcription was used as template in PCR.

Expression of *vpu, vif, tat, nef, rev* and *env* failed to induce significant morphological changes in TE671 cells. *Vpr* expression, on the other hand, induced profound differentiation in the majority of transfected cells.

To verify that TE671 differentiation involves the development of the well-defined muscle phenotype and not a novel program, *vpr*-transfected TE671 cells were stained with an antibody specific for the heavy chain of fast-twitch skeletal-muscle myosin, which is expressed at high levels only in mature skeletal muscle cells. Antibody MY-32 reacted strongly with *vpr*-transfected TE671 cells. The majority of untransfected TE671 cells expressed low levels of myosin, though, as previously reported for TE671, a few untransfected cells stained weakly for myosin. Staining with an isotype-matched control antibody was negative for both transfected and untransfected TE671 cells.

The transfection efficiency achieved by the vpr vector was equal to the efficiency of transfection of the other vectors. Transfection efficiency was determined by the number of cells remaining after selection in puromycin for 2 days, which is sufficient time to kill all nontransfected cells. After several more days, there appeared to be many fewer cells in the *vpr* culture than in the non-*vpr* cultures, owing to the continued replication of the cells in the non-vpr cultures. Not all cells transfected with either the genomic pNLpuro plasmid or with vpr alone underwent morphological differentiation, however. This result is consistent with the heterogeneous response observed in rhabdomyosarcoma lines subjected to differentiation-inducing conditions. More cells remained undifferentiated in the *vpr*-transfected cultures (10%-20%) than in the pNLpuro cultures (<1%). The equal transfection efficiency in the *vpr* culture indicates that *vpr* did not kill replicating cells and leave alive only the naturally occurring spontaneously differentiated cells, which could in theory produce a false interpretation that vpr can induce differentiation. The absolute number of differentiated cells in the HIV *vpr*-transfected cultures was always higher than that found in the other regulatory gene transfections or in untransfected TE671 cells, further indicating that vpr induced differentiation, rather than "revealing" otherwise differentiated cells. Additionally, cells of the radical phenotype observed in the HIV vpr transfected cultures were never observed in the untransfected controls or in the cells transfected with other regulatory genes.

### Other cells are affected by vpr

The *vpr* gene was transfected into the TE671-related rhabdomyosarcoma line RD and the osteogenic sarcoma (osteosarcoma) line D17 to examine the generality of the effects observed in TE671. Following drug selection of the transfected cells, a radical alteration in size and morphology was observed in both cell lines. Inhibition of proliferation was observed in both lines. Time-lapse video microscopy of D17 cells showed them to be very active. The central or perinuclear regions of many cells rotated with a period of approximately 2 hr, frequently resulting in a distinct crescent shape. The majority of the large cells in both cultures remained viable for at least 2 weeks and did not proliferate, though some small proliferating cells remained in both the D17 and RD transfectants, as was observed in the TE671 cultures. The D17 osteosarcoma cells did not express increased levels of alkaline phosphatase, however, which is a marker for bone maturation.

### Deletion of vpr from the HIV-1 Genome

Whether *vpr* is necessary for HIV-1-induced differentiation was next examined. To this end, a *vpr* deletion mutant of the HIV⁻ env plasmid pNLpuro called pNLpuroΔvpr was constructed. A stop codon was introduced after amino acid 22 of *vpr,* and 122 nt were removed from the coding region of *vpr* from amino acid 22 to amino acid 62. First, whether deletion of the vpr gene affected the expression of HIV-1 genes was tested since such an effect might complicate the interpretation of experiments. Viral protein expression following transfection with pNLpuroΔvpr was equal to expression from pNLpuro, as measured by p24^{gag} protein released into the culture medium. Since expression of structural genes by HIV is dependent on successful expression of both tat and rev proteins, it is apparent that the mutation introduced into the genome did not result in a general disturbance of HIV transcription or RNA splicing. As a further control to examine the effect of deletion of vpr on HIV-1 expression, a vpr deletion mutant was constructed from wild-type pNL43, by the same method as that used for the *vpr* deletion mutant of pNLpuro, to yield the env⁺ construct, pNLΔvpr. When transfected into TE671Ψ CD4⁺ transfectant of TE671, syncytia were efficiently produced by both the *vpr⁻* and *vpr*⁺ constructs. Despite nearly equivalent HIV-1 protein production between pNLpuro and pNLpuroΔvpr, the outcome of transfection with the vpr deletion mutant, with respect to differentiation, was clearly different from that of transfection with the *vpr*⁺ HIV1 genome. While cells transfected with pNLpuro differentiated, the majority of the TE671 cells transfected with the *vpr* deletion mutant pNLpuroΔvpr showed either no change or a small and transient increase in size and length. Though a few myosin-staining morphologically differentiated cells were produced in each transfection, the efficiency of this effect varied from experiment to experiment and was never seen to exceed 10% of the cells remaining after drug selection. Taken together these results (summarized in Table 1) demonstrate that HIV-1-induced differentiation of TE671 cells is a function primarily of the vpr gene.

p24^{gag} production in pNLpuroΔvpr continued for 2-3 weeks following transfection and subculture, whereas p24 released from pNLpuro-transfected cells was eliminated following subculture. Subculture effectively eliminated the large differentiated cells, leaving only the replicating undifferentiated cells intact. Therefore, only the differentiated cells released virus in the pNLpuro-transfection experiments. Exposure of the transfectants to the protein kinase C-activating phorbol ester phorbol myristate acetate, which has been shown to stimulate HIV-1 expression in chronically infected cells (Harada, S., *et al*., (1986) *Virology* **154**:249-258), resulted in a 3-fold increase in p24 release from the pNLpuroΔvpr-transfected cells but no measurable p24 release from the undifferentiated pNLpuro-transfected cells. This result indicates that, in the presence of *vpr,* HIV-1 production in TE671 cells is incompatible with their replication, whereas in the absence of *vpr*, HIV1 expression can continue in replicating cells. These cells retain the ability to differentiate in response to various agents and thus remain relatively unaffected by HIV-1 expression.

### Demonstration That Infection with HIV-1 Induces Differentiation

The ability of HIV infection to induce differentiation of the rhabdomyosarcoma cell line was examined. For these experiments, the cell line TE671Ψ was used. TE671Ψ expresses high levels of CD4 on its cell surface and can be infected with HIV at very high efficiency, resulting in a high level of viral production. Infection of TE671Ψ cells at or near confluence results in cell fusion into giant multinucleated syncytia, owing to the fusion of cell membranes following coexpression of HIV envelope proteins and their receptor, CD4. To allow infection and maintenance in culture of unfused cells for several days following infection, TE671Ψ was plated at low cell density, typically 5% confluence or less. Cells plated at low cell density and left unexposed to HIV-1 did not differentiate and continued to replicate. Cells infected with HIV-1 (strains RF or MN) differentiated in a manner very similar to that observed following transfection with the pNLpuro viral genome. These results demonstrate that HIV infection can directly induce cell differentiation.

### Discussion

The unexpected observation that transfection of HIV-1 genomic DNA into the embryonal rhabdomyosarcoma line TE671 induced cell growth inhibition and differentiation is reported here. Infection of TE671 via a transfected CD4 molecule resulted in the same outcome, indicating that the effects did not result from transfection artifacts and have relevance to natural HIV infection. Transfection and expression of each regulatory gene of HIV-1 in the cell line revealed that the vpr gene can produce the growth inhibition and morphological differentiation that the whole virus induces. Activation of the endogenous muscle program was demonstrated by showing that the *vpr-*transfected cells expressed high levels of fast-twitch myosin, while the majority of untransfected cells did not. Transfection of a *vpr* deletion mutant into TE671 cells resulted in the production of large numbers of replicating undifferentiated cells that continued to produce high levels of viral protein. These results indicate that *vpr* is the primary determinant for differentiation and growth inhibition in TE671 cells. Transfection of *vpr* into the rhabdomyosarcoma line RD and the osteosarcoma line D17 resulted in cessation of proliferation, gross morphological changes, and profound enlargement. Thus *vpr* may be a regulator of cell function in cells of diverse origin.

Muscle differentiation has been well studied in rhabdomyosarcomas and in normal cells. Expression of helix-loop-helix transcription factors such as MyoD in normal myoblasts leads to differentiation into mature postmitotic myotubes. In most embryonal rhabdomyosarcomas, despite expression of MyoD, withdrawal from the cell cycle and differentiation are inhibited. Transformation of embryonal rhabdomyosarcomas is linked to expression of an activated ras oncogene, loss of a putative tumor suppressor on chromosome 11, and constitutive expression of autocrine fibroblast growth factor and transforming growth factor *β*. In addition, RD (and therefore TE671) has been shown to lack a wild-type p53 tumor suppressor gene. p53 expression has recently been associated with cell cycle control and the regulation of DNA repair mechanisms, cellular events linked to retroviral integration. Osteosarcomas exhibit features of bone matrix-secreting osteoblasts but are thought to arise from multipotential mesenchymal tissue and therefore to represent similarly a disregulation of primitive cells. These tumors also typically display loss-of-function p53 mutations. *Vpr* can at least partially overcome the block on differentiation and completely restore inhibition of cell proliferation; therefore, *vpr* may either replace a function lost during transformation or activate a pathway that over-rides the genetic defects.

These studies directly demonstrate that the HIV-1 *vpr* gene encodes a protein that can function in the regulation of basic cellular events. The outcome of this regulation is observed here as an inhibition of cell proliferation and the induction of differentiation.

### Example 2

A pharmaceutical composition is formulated by providing 100µg/µl pBabe-puro+vpr combined with sterile phosphate buffered saline that is isotonic with cells. The composition is administered by direct injection into a solid tumor mass of an individual.

### Example 3

DNA encoding HIV-1 vpr protein is amplified via PCR from the HIV-1 genomic plasmid pNL43 using the PCR primers and strategy described in Example 1. The resulting PCR product is inserted into expression vector plasmid pSE420 (Invitrogen, San Diego, CA) and introduced into *E. coli.*

A pharmaceutical composition is prepared by isolating vpr protein form the cells and/or medium and combining it with a sterile pharmaceutically acceptable solution.

### Example 4

DNA encoding HIV-1 *vpr* protein is amplified via PCR from the HIV-1 genomic plasmid pNL43 using the PCR primers and strategy described in Example 1. The resulting PCR product is inserted into expression vector plasmid pYES2 (Invitrogen, San Diego, CA) and introduced into S. cerevisiae.

A pharmaceutical composition is prepared by isolating *vpr* protein form the cells and/or medium and combining it with a sterile pharmaceutically acceptable solution.

### Example 5

DNA encoding HIV-1 *vpr* protein is amplified via PCR from the HIV-1 genomic plasmid pNL43 using the PCR primers and strategy described in Example 1. The resulting PCR product is inserted into expression vector plasmid pcDNA I (Invitrogen, San Diego, CA) and introduced into Chinese Hamster Ovary (CHO) cells.

A pharmaceutical composition is prepared by isolating vpr protein from the cells and/or medium and combining it with a sterile pharmaceutically acceptable solution. **Example 6**

Recombinant vpr protein was produced in baculovirus. Production of recombinant vpr protein allowed for studies of the function of the protein in vitro, permitted the generation of anti-*vpr* antibodies in rabbits and monoclonal antibodies in mice.

The *vpr* gene was cut out of the *vpr*-pBabe-puro construct. This insert was then introduced into the baculovirus expression vector pVL-1393 (PharMingen) by standard techniques. Recombinant viruses were produced as previously described (Matsuura et al. 1987) using Baculogold (PharMingen) linearized DNA in cotransfection experiments into *Spodoptera fungupeida* (SF-9) cells. SF-9 cells and the subsequent viral infections were carried out as previously described (Matsuura et al. 1987, O'Reilly et al. 1991). The presence of recombinant virus was easily observed under the light microscope following transfection. The presence of vpr protein was tested by Enzyme linked immunosorbent assay (ELISA) and western Blot analysis, using a rabbit anti-peptide (amino acids 2-21 N-terminus) polyclonal serum obtained through the AIDS repository.

Sf-9 cells were infected with recombinant virus at a multiplicity of infection of 5-10 and harvested at various times post infection. Whole cell and supernatant fractions were analyzed by ELISA using the aforementioned antibody. As early as 24 hours post infection, recombinant protein could be found in the supernatant. This presence would reach its peak at 30 hrs. post infection. Fractionation experiments were undertaken to optimize the collection of vpr protein. The majority of the *vpr* reactivity was found to be located in supernatant of recombinant Sf-9 cells. Little additional vpr protein was recovered from nonionic detergent lysates of cell sonicates.

The activity of this protein was further verified by screening the protein containing cell culture supernatant with a mixture of HIV+ seropositive patient samples as well as control samples. HIV patients have been reported to produce an humoral immune response to the *vpr* gene product. Immulon II ELISA plates (Dynatech laboratories, Chantilly, Virginia) were coated with three dilutions of the *vpr* supernatant. These were then probed with 2 serial dilutions of heat inactivated patient sera. Our results demonstrate that HIV positive patient sera did react with the *vpr* present in the cell supernatant. The level of their reactivity was not correlated to the levels of reactivity the sera contained against the HIV-1 envelope (as measure by solid phase ELISA).

Rabbit anti-vpr protein was titrated to 1/1000 dilution and used in ELISA assay to determine production of *vpr* by various Sf-9 cell preparations. Specific and significant reactivity was observed in supernatant fraction from vpr transfected cells only. The rabbit antisera is an epitope restricted anti-peptide antisera and as such may not recognize a poorly processed insoluble cell associated *vpr* fraction in these cells. To confirm vpr production the identical samples were reacted with pooled HIV patient sera and pooled normal human sera was used as a specific control. HIV positive patient sera reacted with the supernatant containing fraction in a similar manner to the rabbit antisera. These same sera samples reacted very poorly with supernatants from non-infected Sf-9 cells. The same patient sera reacted poorly with supernatants from Sf-9 cells which were infected with control recombinant viruses (viruses which were formed by cotransfecting linearized DNA and pVL 1393 alone). The reactivity observed demonstrates the production of *vpr* protein in the supernatants of Sf-9 infected cells.

The vpr protein containing supernatant was subjected to purification by two different chromatography methods.

Supernatants from infected Sf-9 cells were concentrated in an amicon pressure filter unit, dialyzed, clarified and treated with protease inhibitors prior to column chromatography. The 24 hour vpr product in the supernatant was concentrated, centrifuged at 10000g for 10 min. Protease inhibitor were then added to this supernatant (PMSF, aprotonin, leupeptin and EDA) at their appropriate concentrations. This solution was then passed over a protein A-rabbit anti-*vpr* column. Rabbit anti-*vpr* immunoglobulin was purified on a protein A-agarose column and eluted and dialyzed and then coupled to CNBr-sepharose 4B beads according to the manufacturers instructions (Sigma). This column was then utilized for immunoaffinity chromatography of baculovirus vpr. *Vpr* was eluted in a pH gradient. The column was then washed with PBS. 10mM Na-Phosphate, ph 8.0, and elution by a pH gradient was undertaken. Specific reactivity appeared to be concentrated over a limited fraction number as determined by rabbit anti-*vpr* antisera reactivity in ELISA. The specific protein peak and activity peak clearly overlap.

In addition, *vpr* protein was collected off a DEAE sepharose column using a salt gradient. Baculovirus-*vpr* protein containing supernatant was treated as above and then placed over a DEAE-sepharose column. The column was eluted by salt gradient, vpr activity was concentrated over a limited range.

Both purification procedures generate samples which react with HIV patient samples as well as the rabbit anti-*vpr* peptide antisera in ELISA and in western blotting experiments. In western blotting experiments, a 26Kd protein is present a dominant 14Kd protein and two small protein bands suggestive of breakdown products are observed. The 26Kd band may represent an artifact of purification (such as acetylation) or may indicate a state of aggregation requiring further investigation.

### Example 7

DNA encoding HIV-1 vpr protein was amplified via PCR from the HIV-1 genomic plasmid pNL43 using the PCR primers and strategy described in Example 1. The resulting PCR product is inserted into expression vector plasmid of the MaxBac^{™} (Invitrogen, San Diego, CA) complete baculovirus expression system and introduced into insect cells used in that system.

A pharmaceutical composition is prepared by isolating vpr protein form the cells and/or medium and combining it with a sterile pharmaceutically acceptable solution.

### Example 8

Glioblastoma cell lines were tranfected with the *vpr* expression vector and specific examples of differentiation events were observed. For differentiation experiments the cells were transfected either by a lipid-mediated method using DOTAP (Boehringer Mannheim). DOTAP (Boehringer Mannheim) transfection was performed per the manufacturer's instructions in tissue culture flasks on 0.5-1 x 10⁶ cells in log phase growth. Selection medium was added 48-60 hours post transfection and cells were maintained in selection for the duration of the experiments. Cells transfected with plasmids containing the puromycin resistance gene (PAC) were selected in 1 µg/ml puromycin (Sigma) For both the cell lines U87-MG and U138 MG specific morphological differentiation was observed. In comparison to control transfected cells the vpr transfected cells exhibited extended pseudopods and demonstrated neurite outgrowth concurrent with an observed inhibition of their cellular proliferation. Both cell lines also demonstrated an increased cytoplasmic to nuclear ratio as well as a clear enlargement in cell size. Frequent bipolarity was also observed in the vpr transfected cell lines along with active cytoskeletal activity.

Test assays comprise the steps of adding test compound to the to the medium used in the cell cultures. Test compound is provided in 10 dilutions ranging from 10µM to 100µM.

A control assay may optionally be run comprising the step of adding vpr protein to cells without adding test compound.

Analysis of non-specific and lineage specific markers in these cell lines is performed 2 to 14 days post transfection.

### Example 9

A screen for identifying compounds which inhibit *vpr*'s ability to induce differentiation of undifferentiated cells is performed as follows.

Either the human embryonal rhabdomyosarcoma TE671 line (ATCC HTB 139) and the canine osteosarcoma D17 line (ATCC CLL 183) are used. The cells are maintained in appropriate cell culture medium under standard conditions.

*Vpr* is produced as described in Examples 3, 4, 5 or 6.

Test assays comprise the steps of contacting the cells with *vpr* in the presence of a test compound. A mixture of *vpr* and the test compound are added to the cell culture medium together or separately. Test compound is provided in 10 dilutions ranging from 10µM to 100µM.

A control assay may optionally be run comprising the step of adding vpr protein to cells without adding test compound.

After 2 - 14 days, cells are observed to determine whether differentiation has occurred. Morphological and size changes indicating differentiation are described in Example 1. Visual observation may be accompanied by or substituted with an antibody assay to observed whether myosin is being produced by the cells. Anti-myosin assay is performed as described in Example 1.

### Example 10

A simple in vitro ELISA based system for mapping interaction sites between *vpr* and *gag* p55. Baculovirus produced *vpr* as outlined above and baculovirus gag produced by similar means were used in binding assays. ELISA plates were coated with gag or vpr and reacted with specific antisera as controls, or coated with dilutions of *gag* protein followed by *vpr* and sandwiched with anti-*vpr* antibodies. Alternatively, plates were coated with dilutions of *vpr* followed by *gag* protein and sandwiched with anti-*gag* specific antisera. Controls for specificity include that *gag* antisera does not react with *vpr, vpr* antisera does not react with gag, neither *gag* nor *vpr* antisera reacts with BSA. Plates were coated with recombinant antigen in carbonate buffer, washed extensively, blocked with PBS/1% BSA and then washed extensively, secondary protein was dissolved in PBS/BSA and incubated at 4C 1hr, then washed extensively and reacted with specific antisera. Specific sandwich activity was detected in both directions as described above.

### Example 11

Using PCR and recombinant DNA technology, truncation mutants of the vpr gene were constructed and cloned into pBABE expression plasmids. These constructs delete vpr in approximately 20AA groups from the carboxy terminus traveling in toward the amino terminus of the protein. The resulting protein products are 72AA, 50AA and 30AA.

Preliminary studies indicate that the carboxyl terminus 24AA of *vpr* is necessary for induction of differentiation of both the rhabdomyosarcoma and glial cell lineages as loss of the inhibition of proliferation and loss of morphological changes with the deletion mutants has been observed. One interesting observation of these studies is that this carboxy region contains a significant region of homology with the muscle oncogene ski. The avian retroviral oncogene ski shows properties resembling those described for *vpr* (Colmenares and Stavnezer, Cell, 1989).

Studies suggest that carboxy terminal deletion vpr mutants still retain gag binding activity in this system. This assay therefore differentiates the functional region of *vpr* which interacts with gag and the functional region for cell differentiation function.

### Example 12: Antibodies and Immunizations

Rabbit anti-*vpr* peptide serum (Garrett, *et al., J. Virol.,* **1991**, *65*, 1653) (a.a. 2-21: Cys-Glu-Gln-Ala-Pro-Glu-Asp-Gln-Gly-Pro-Gln-Arg-Glu-Pro-His-Asn-Glu-Trp-Thr-Leu-Glu; SEQ ID NO:4) was obtained from Dr. Brian Cullen through the NIH AIDS Research and Reference Reagent Program. To produce additional rabbit antibodies against vpr, a rabbit was immunized with 10-20 µg of partially purified *vpr* protein (produced as described below from the anti-*vpr* column) in complete Freund's adjuvant (CFA) once, then with incomplete adjuvant (IFA) for subsequent immunizations. Final immunization was with 50 µg of each of three keyhole limpet hemocyanin (KLH)-coupled vpr peptides in IFA. Peptides were purchased from American Bio-Technologies. Sequences of peptides: vpr 9-20 (Gly-Pro-Gln-Arg-Glu-Pro-His-Asn-Glu-Trp-Thr-Leu; SEQ ID NO:5), 41-55 (Gly-Leu-Gly-Gln-His-Ile-Tyr-Glu-Thr-Gly-Asp-Thr-Trp-Ala; SEQ ID NO:6), 81-96 (Ile-Gly-Val-Thr-Gln-Gln-Arg-Arg-Gln-Arg-Asp-Gly-Ala-Ser-Arg-Ser; SEQ ID NO:7). To produce mouse anti-*vpr* serum, Balb-c mice were immunized with 20 µg of single peptides coupled to KLH in CFA for the first immunization and IFA for subsequent immunizations.

### Example 13: Column Chromatography

Affinity columns were constructed according to Harlow and Lane. Harlow, E. and Lane, E., *Antibodies: A Laboratory Manual*, **1988**, Cold Spring Harbor Laboratory Press which is incorporated herein by reference. The IgG fraction of 250 µl of the rabbit peptide serum was bound to 1 ml of protein A agarose beads (Gibco BRL), washed in 0.2 M sodium borate buffer (pH 9.0) and coupled with 20 mM dimethylpimelimidate (DMP). The polyclonal rabbit anti-*vpr* column was constructed according to the same procedure using 6 ml of serum and 3 ml of protein G agarose beads (Gibco BRL).

### Example 14: Detection of Anti-vpr Antibodies by Capture ELISA

For detection of anti-*vpr* antibodies, an ELISA was performed using eukaryotically-produced *vpr* attached to solid phase, followed by the addition of the test sample. Peroxidase-coupled anti-human antibody was used for detection (Boehringer Mannheim). Color development was with 3,3',5,5'-tetramethylbenzidine dihydrochloride (TMB) (Sigma) according to the manufacturer's instructions. Anti-p24 antibodies were detected using recombinant p24 (American BioTechnologies) attached to solid phase support. Both recombinant proteins were used at an approximate concentration of 1 µg/ml, 50 µl/well. Incubation was done for 1 hour at 37° or 12 hours at 4°. Detection antibodies were used at a 1:15000 dilution as per manufacturers directions.

### Example 15: Detection of vpr by Capture ELISA

For detection of *vpr,* a capture ELISA was performed. Rabbit anti-*vpr* peptide serum (reactive to aa 2-21) was immobilized in wells of a 96-well ELISA plate (Immulon II, Dynatech) in carbonate-bicarbonate buffer (0.2 M, pH 9.2). Detection of bound antigen was performed using a mouse anti-*vpr* peptide serum (reactive to aa 81-96) followed by peroxidase coupled anti-mouse antibody (Boehringer Mannheim). Color development was with TMB as described previously. The rabbit antibody was used at 1:1000, the mouse antibody was used at 1:800. Incubation was done for 1 hour at 37° or 12 hours at 4°. Anti-mouse antibodies were used at a 1:12000 dilution as per manufacturers directions.

### Example 16: Production of Eukaryotic vpr.

To construct a recombinant baculovirus containing the *vpr* gene, the *vpr* open reading frame and a PCR-introduced consensus eukaryotic ribosome binding sequence, from the vpr-pBabe-puro expression plasmid previously described (Levy *et al., Cell,* **1993,** *72,* 541) and incorporated herein by reference, was subcloned into the multiple cloning site of the pVL1393 baculovirus vector (Invitrogen) downstream of the baculovirus polyhedron promoter by standard techniques known to those skilled in the art. This construct is predicted to encode a non-fused, native vpr protein. Transfection of this plasmid along with linearized AcMNPV genomic DNA (BaculoGold, PharMingen) by standard techniques into SF9 (*Spodoptera frugiptera*) insect cells yielded recombinant baculoviruses containing the *vpr* gene. Twenty four hours after transfection, virus-containing supernatants from transfected cells were applied to new High Five cells (*Trichoplusia ni*) whose supernatants and cell fractions were then assayed for *vpr* protein expression by standard techniques. *Vpr* protein was detected in the supernatants and in the cell fractions of infected cells by ELISA within 12 hours of infection and not in the supernatants or cell fractions of cells infected with baculovirus, prepared identically to the vpr recombinants but lacking a vpr gene. Peak vpr levels in the supernatant were detected at 24 hours post-infection. The reasons for the preferential export of *vpr* into the baculovirus supernatants is not known. However, export of recombinant proteins is not unusual for this expression system.

### Example 17: Partial Purification of vpr Protein.

*Vpr*-containing supernatants were applied to an anti-*vpr*-peptide affinity column, constructed by standard methods. Partial purification and concentration was achieved by triethanolamine (pH 11.5) elution followed by DEAE sepharose chromatography by techniques known to those skilled in the art. This material displayed both the monomeric and putative homodimeric forms of vpr with roughly ten times more monomer than dimer observed. The material was used to immunize a rabbit 3 times at 6-8 week intervals, followed by a single immunization with 3 vpr peptides representing amino and carboxyl terminal residues and a central hydrophilic portion of the molecule. The final immunization with vpr peptides served to increase the specific anti-*vpr* titer of the serum. The resulting serum bound native viral and recombinant baculovirus vpr proteins in Western blot and ELISA and specifically recognized the 3 vpr peptides in ELISA. Optimum dilution of serum for ELISA was 1:10,000. No cross-reactivity with other components of the baculovirus supernatant or to any other proteins was observed by Western blot or ELISA. An affinity column was prepared using this serum and vpr supernatants were applied. The protein eluting from this column displayed three major bands on silver stained or coomassie stained SDS-PAGE gels, representing the monomer and dimer forms of vpr, plus a 50 kDa band which probably represents the lactalbumin present in the cell growth medium nonspecifically bound to the column.

### Example 18: Biological Activity of Baculovirus vpr.

Recombinant *vpr* protein can be tested for biological activity in tissue culture. *Vpr* and control supernatants were dialyzed against PBS and applied to TE671 rhabdomyosarcoma cells. TE671 cells differentiate and cease to proliferate when transfected with the vpr gene or infected with HIV (Gras-Masse *et al., supra*; Levy *et al., supra*). Surprisingly, TE671 cells exposed continuously to 20% vpr supernatant ceased proliferation after approximately 3 days and after 7-9 days underwent morphological differentiation similar to that observed following transfection with the HIV-1 *vpr* gene, including great enlargement, the presence of long processes similar to myotubes, multinucleation and cessation of proliferation. Exposure to greater than 20% supernatant proved to be rapidly fatal to the cells. Incubation of TE671 cells with equal concentrations of control supernatants failed to induce any of these changes. Cells exposed to vpr for 3 days were examined for the presence of adult muscle myosin, a differentiation marker for these cells (Aguanno *et al*., *Cancer Res.,* **1990,** *50,* 3377). Greater than 90% of cells stained positive using an anti-myosin antibody, demonstrating that the differentiation was along the pathway to which to these cells are committed and is identical to that induced by expression of *vpr* from within.

### Example 19

Melanoma cells were transfected with a nucleic acid molecules that comprised a nucleotide sequence that encoded vpr. In test experiments, transfected cells were selected and implanted into mice. As a control, untransfected melanoma cells were introduced into other mice.

The mice with implanted transfected melanoma cells displayed a vast reduction in the number of tumors developing from the injected cells as compared to the number of tumors developing in control mice. The results from these experiments clearly demonstrated that while *vpr* did not complete eliminate the tumorigenicity of the melanoma cells, the presence of the *vpr* gene in the melanoma cells significantly and substantially reduced the tumorigenicity of the cells.

| **Table 1. HIV-1 Constructs Used in Example 1** | | | | | |
|---|---|---|---|---|---|
| Construct | Vector or Derivation | vpr | Differentiation | p24 Production | Characteristics |
| pNLpuro | HIV1 pNL43 | + | +++ | +++ | *env* deletion mutant |
| *vpr* | pBABE-puro | + | +++ | N/A | |
| *vif* | pBABE-puro | - | - | N/A | |
| *vpu* | pBABE-puro | - | - | N/A | |
| *nef* | pBABE-puro | - | - | N/A | |
| *tat* | pCV-1 | - | - | N/A | |
| *env-rev* | pcDNAl/neo | - | - | N/A | *env*^{±}, syncytia:++ |
| pNLpuroΔvpr | pNL43 | - | -/+^{a} | +++ | *env,* deletion mutant |
| pNL43 | pNL43 | + | NT | +++ | *env*^{±}, syncytia in TE671Ψ:++ |
| pNLΔvpr | pNL43 | - | NT | +++ | *env*^{±}, syncytia in TE671Ψ:++ |
| p24 release into the culture medium was measured 48 hr following transfection, as described in Experimental Procedures. ^{a}Transient increase in size in some cells, with reversion of phenotype observed after approximately 6 days, and differentiation in a small subset (5%-10%) NT, not tested; N/A, not applicable. | | | | | |

## Claims

1. A method of inducing undifferentiated cells to differentiate which comprises the steps of:
contacting undifferentiated cells *in vitro* with an amount of HIV vpr protein or a functional fragment thereof effective to stimulate differentiation; or
introducing into undifferentiated cells *in vitro* a nucleic acid molecule that comprises a nucleotide sequence that encodes HIV vpr protein or a functional fragment thereof whereby said nucleotide sequence is expressed by said cells,
wherein the undifferentiated cells are not human embryonic stem cells.

2. A method according to claim 1, wherein the undifferentiated cells are tumour cells.

3. A pharmaceutical composition comprising:
a) HIV vpr protein or a functional fragment thereof, or a nucleic acid molecule that comprises a nucleotide sequence that encodes HIV vpr protein or a functional fragment thereof; and
b) pharmaceutically acceptable carrier.

4. The pharmaceutical composition according to claim 3, wherein said pharmaceutical composition is adapted to use in an intratumoral injection.

5. The pharmaceutical composition according to claim 3, wherein said pharmaceutical composition is adapted for topical administration.

6. The use of HIV vpr protein or a functional fragment thereof or a nucleic acid molecule that comprises a nucleotide sequence that encodes HIV vpr protein or a functional fragment thereof in the manufacture of a medicament for treating an individual diagnosed with or suspected of suffering from a disease associated with hyperproliferating undifferentiated cells.

7. The use of HIV vpr protein or a functional fragment thereof or a nucleic acid molecule that comprises a nucleotide sequence that encodes HIV vpr protein or a functional fragment thereof in the manufacture of a medicament for treating an individual diagnosed with or suspected of suffering from cancer.

8. The use according to claim 7, wherein said cancer is **characterized by** a solid tumor.

9. The use according to claim 8 wherein the medicament is adapted for use in an intratumoral injection.

10. The use according to claim 6, wherein said disease is psoriasis.

11. The use according to claim 10, wherein said medicament is adapted for topical administration.

12. A method of identifying compounds which inhibit HIV vpr from stimulating differentiation of undifferentiated cells which comprises the steps of
a) contacting, in the presence of a test compound, said undifferentiated cells with an amount of HIV vpr protein sufficient to stimulate differentiation and
b) comparing the differentiation that occurs with the differentiation that occurs when said undifferentiated cells are contacted with HIV vpr protein in the absence of said test compound,
wherein the undifferentiated cells are not human embryonic stem cells.

13. A kit for performing the method of identifying compounds which inhibit HIV vpr from stimulating differentiation of undifferentiated cells of claim 12, said kit comprising
a) a first container comprising undifferentiated cells, and
b) a second container comprising HIV vpr protein,
wherein the undifferentiated cells are not human embryonic stem cells.

## Patentansprüche

1. Verfahren, um bei undifferenzierten Zellen die Differenzierung zu induzieren, das folgende Schritte aufweist:
In-Kontakt-Bringen von undifferenzierten Zellen in vitro mit einer Menge des HIV-vpr-Proteins oder eines funktionellen Fragmentes davon, die ausreichend ist, um die Differenzierung zu stimulieren; oder
Einbringen eines Nucleinsäuremoleküls, das eine Nucleotidsequenz aufweist, die für das HIV-vpr-Protein oder ein funktionelles Fragment davon codiert, in undifferenzierte Zellen *in vitro,* wobei die Nucleotidsequenz von den Zellen exprimiert wird, wobei die undifferenzierten Zellen nicht humane embryonale Stammzellen sind.

2. Verfahren gemäß Anspruch 1, wobei es sich bei den undifferenzierten Zellen um Tumorzellen handelt.

3. Pharmazeutische Zusammensetzung, die Folgendes aufweist, nämlich:
a) ein HIV-vpr-Protein oder ein funktionelles Fragment davon oder ein Nucleinsäuremolekül, das eine Nucleotidsequenz aufweist, die für das HIV-vpr-Protein oder ein funktionelles Fragment davon codiert; und
b) einen pharmazeutisch akzeptablen Träger.

4. Pharmazeutische Zusammensetzung gemäß Anspruch 3, wobei die pharmazeutische Zusammensetzung zur Verwendung in einer intratumoralen Injektion ausgestaltet ist.

5. Pharmazeutische Zusammensetzung gemäß Anspruch 3, wobei die pharmazeutische Zusammensetzung zur topischen Verabreichung ausgestaltet ist.

6. Verwendung eines HIV-vpr-Proteins oder eines funktionellen Fragmentes davon oder eines Nucleinsäuremoleküls, das eine Nucleotidsequenz aufweist, die für ein HIV-vpr-Protein oder ein funktionelles Fragment davon codiert, zur Herstellung eines Arzneimittels zur Behandlung eines Individuums, für das eine Diagnose oder ein Verdacht für ein Krankheitsleiden vorliegt, das im Zusammenhang steht mit der Hyperproliferation von undifferenzierten Zellen.

7. Verwendung eines HIV-vpr-Proteins oder eines funktionellen Fragmentes davon oder eines Nucleinsäuremoleküls, das eine Nucleotidsequenz aufweist, die für ein HIV-vpr-Protein oder ein funktionelles Fragment davon codiert, zur Herstellung eines Arzneimittels zur Behandlung eines Individuums, für das eine Diagnose oder ein Verdacht für ein Krebsleiden vorliegt.

8. Verwendung gemäß Anspruch 7, wobei der Krebs durch einen festen Tumor charakterisiert ist.

9. Verwendung gemäß Anspruch 8, wobei das Medikament zur Verwendung in einer intratumoralen Injektion ausgestaltet ist.

10. Verwendung gemäß Anspruch 6, wobei es sich bei der Krankheit um Psoriasis handelt.

11. Verwendung gemäß Anspruch 10, wobei das Arzneimittel zur topischen Verabreichung ausgestaltet ist.

12. Verfahren zur Identifizierung von Komponenten, die das HIV-vpr in der Stimulierung der Differenzierung von undifferenzierten Zellen inhibieren, das folgende Schritte aufweist:
a) In-Kontakt-Bringen der undifferenzierten Zellen in Gegenwart einer Testkomponente, mit einer Menge von HIV-vpr-Protein, die ausreichend ist, um die Differenzierung zu stimulieren, und
b) Vergleichen der stattfindenden Differenzierung, mit der Differenzierung, die stattfindet, wenn die undifferenzierten Zellen in Abwesenheit der Testkomponente mit dem HIV-vpr-Protein in Kontakt gebracht werden, wobei die undifferenzierten Zellen nicht humane embryonale Stammzellen sind.

13. Kit zur Durchführung des Verfahrens zur Identifizierung von Komponenten, die das HIV-vpr in der Stimulierung der Differenzierung von undifferenzierten Zellen inhibieren, gemäß Anspruch 12, wobei das Kit Folgendes aufweist, nämlich:
a) ein erstes Behältnis, das undifferenzierte Zellen aufweist, und
b) ein zweites Behältnis, das das HIV-vpr-Protein aufweist, wobei die undifferenzierten Zellen nicht humane embryonale Stammzellen sind.

## Revendications

1. Procédé pour induire des cellules non différenciées pour effectuer une différenciation, qui comporte les étapes dans lesquelles :
on met en contact des cellules non différenciées in vitro avec une quantité de protéine vpr (Protéine Virale R) HIV ou de l'un de ses fragments fonctionnels qui est efficace pour stimuler la différenciation ; ou
on introduit dans des cellules non différenciées in vitro une molécule d'acide nucléique qui comporte une séquence de nucléotide qui code la protéine vpr HIV ou l'un de ses fragments fonctionnels, la séquence de nucléotide étant exprimée par les cellules,
les cellules non différenciées n'étant pas des cellules souches d'embryon humain.

2. Procédé suivant la revendication 1, dans lequel les cellules non différenciées sont des cellules tumorales.

3. Composition pharmaceutique comportant :
a) une protéine vpr HIV ou l'un de ses fragments fonctionnels, ou une molécule d'acide nucléique qui comporte une séquence de nucléotide qui code la protéine vpr HIV ou l'un de ses fragments fonctionnels ; et
b) un support pouvant être accepté d'un point de vue pharmaceutique.

4. Composition pharmaceutique suivant la revendication 3, dans laquelle la composition pharmaceutique est conçue pour une utilisation dans une injection intratumorale.

5. Composition pharmaceutique suivant la revendication 3, dans laquelle la composition pharmaceutique est conçue pour une administration topique.

6. Utilisation de la protéine vpr HIV ou de l'un de ses fragments fonctionnels ou d'une molécule d'acide nucléique qui comporte une séquence de nucléotide qui code la protéine vpr HIV ou de l'un de ses fragments fonctionnels dans la fabrication d'un médicament pour traiter un individu auquel a été diagnostiqué une maladie associée à l'hyperprolifération de cellules non différenciées ou qui est suspecté de souffrir d'une telle maladie.

7. Utilisation de protéine vpr HIV ou d'un de ses fragments fonctionnels ou d'une molécule d'acide nucléique qui comporte une séquence de nucléotide qui code une protéine vpr HIV ou un de ses fragments fonctionnels dans la fabrication d'un médicament pour traiter un individu à qui on a diagnostiqué un cancer ou qui est suspecté de souffrir d'un cancer.

8. Utilisation suivant la revendication 7, dans laquelle le cancer est **caractérisé par** une tumeur solide.

9. Utilisation suivant la revendication 8, dans laquelle le médicament est conçu pour une utilisation suivant une utilisation intratumorale.

10. Utilisation suivant la revendication 6, dans laquelle la maladie est le psoriasis.

11. Utilisation suivant la revendication 10, dans laquelle le médicament est conçu pour une administration topique.

12. Procédé d'identification de composés qui inhibent la vpr HIV à partir de la stimulation de la différenciation de cellules non différenciées qui comporte les étapes dans lesquelles :
a) on met en contact, en la présence d'un composé de test, les cellules non différenciées avec une quantité de protéine vpr HIV suffisante pour stimuler une différenciation et
b) on compare la différenciation qui a lieu avec la différenciation qui a lieu lorsque les cellules non différenciées sont mises en contact avec une protéine vpr HIV en l'absence du composé de test,
les cellules non différenciées n'étant pas des cellules souches d'embryon humain.

13. Trousse pour mettre en oeuvre le procédé d'identification de composés qui inhibent la vpr HIV pour l'empêcher de stimuler une différenciation de cellules non différenciées suivant la revendication 12, la trousse comportant
a) un premier récipient comportant des cellules non différenciées, et
b) un deuxième récipient contenant une protéine vpr HIV,
les cellules non différenciées n'étant pas des cellules souches d'embryon humain.


### SEQUENCE LISTING

(1) GENERAL INFORMATION:
   (i) APPLICANT:
      (A) NAME: David B WEINER
      (B) STREET: 717 Beacom Lane
      (C) CITY: Merion
      (D) STATE: Pennsylvania
      (E) COUNTRY: USA
      (F) POSTAL CODE (ZIP): PA 19066

      (A) NAME: David Nathan LEVY
      (B) STREET: 24 Queensbury Street, Apartment 11
      (C) CITY: Boston
      (D) STATE: Massachussetts
      (E) COUNTRY: USA
      (F) POSTAL CODE (ZIP): MA 02215

      (A) NAME: Yosef REFAELI
      (B) STREET: 88 Washington Street, Apartment 27
      (C) CITY: Boston
      (D) STATE: Massachussetts
      (E) COUNTRY: USA
      (F) POSTAL CODE (ZIP): MA 02135
   (ii) TITLE OF INVENTION: VPR function and activity
   (iii) NUMBER OF SEQUENCES: 10
   (iv) COMPUTER READABLE FORM:
      (A) MEDIUM TYPE: Floppy disk 1.44M
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      APPLICATION NUMBER: EP 94909839.6
(2) INFORMATION FOR SEQ ID NO: 1:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 1:
      GGCGGCTCGA GGATCCGCCG CCACCATG 28
(2) INFORMATION FOR SEQ ID NO: 2:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 2:
      GGGGCTTGTT CCATGGTGGC 20
(2) INFORMATION FOR SEQ ID NO: 3:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 3:
      CCGCGGATCC TAGGATCTAC TGGC 24
(2) INFORMATION FOR SEQ ID NO: 4:
   (i) SEQUENCE CHARACTERISTICS:
      (A) LENGTH: 21 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 4:
   Cys Glu Gln Ala Pro Glu Asp Gln Gly Pro Gln Arg Glu Pro His Asn
   1               5                   10                  15
   Glu Trp Thr Leu Glu
               20
(2) INFORMATION FOR SEQ ID NO: 5:
   (i) SEQUENCE CHARACTERISTICS:
      (A) LENGTH: 12 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 5:
   Gly Pro Gln Arg Glu Pro His Asn Glu Trp Thr Leu
   1               5                   10
(2) INFORMATION FOR SEQ ID NO: 6:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 6:
   Gly Leu Gly Gln His Ile Tyr Glu Thr Gly Asp Thr Trp Ala
   1               5                   10
(2) INFORMATION FOR SEQ ID NO: 7:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 7:
   Ile Gly Val Thr Gln Gln Arg Arg Gln Arg Asp Gly Ala Ser Arg Ser
   1               5                   10                  15
(2) INFORMATION FOR SEQ ID NO: 8:
   (i) SEQUENCE CHARACTERISTICS:
      (A) LENGTH: 12 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 8:
   Gly Pro Gln Arg Glu Pro His Asn Glu Trp Thr Leu
   1               5                   10
(2) INFORMATION FOR SEQ ID NO: 9:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 9:
   Gly Leu Gly Gln His Ile Tyr Glu Thr Tyr Gly Asp Thr Trp Ala
   1               5                   10                  15
(2) INFORMATION FOR SEQ ID NO: 10:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 10:
   Ile Gly Val Thr Gln Gln Arg Arg Ala Arg Asn Gly Ala Ser Arg Ser
   1               5                   10                  15